(19) Europäisches Patentamt / European Patent Office / Office européen des brevets

(11) **EP 4 417 305 A1**

(12) **EUROPEAN PATENT APPLICATION**
published in accordance with Art. 153(4) EPC

(43) Date of publication:
**21.08.2024 Bulletin 2024/34**

(21) Application number: **22880939.8**

(22) Date of filing: **07.10.2022**

(51) International Patent Classification (IPC):
**B01J 29/18** (2006.01)    **B01J 35/10** (2006.01)
**B01J 37/04** (2006.01)    **B01J 37/06** (2006.01)
**B01J 37/08** (2006.01)    **C01B 39/26** (2006.01)
**C07B 61/00** (2006.01)    **C07C 209/14** (2006.01)
**C07C 211/04** (2006.01)

(52) Cooperative Patent Classification (CPC):
**B01J 29/18; B01J 35/60; B01J 37/04; B01J 37/06;
B01J 37/08; C01B 39/26; C07B 61/00;
C07C 209/14; C07C 211/04;** Y02P 20/584

(86) International application number:
**PCT/JP2022/037566**

(87) International publication number:
**WO 2023/063244 (20.04.2023 Gazette 2023/16)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **11.10.2021 JP 2021166985**

(71) Applicant: **Resonac Corporation
Tokyo 105-7325 (JP)**

(72) Inventors:
• **UEDA, Yasuyuki
Tokyo 105-7325 (JP)**

• **NAKAMURA, Keiichi
Tokyo 105-7325 (JP)**
• **UCHIDA, Hiroshi
Tokyo 105-7325 (JP)**
• **MATANO, Akihiro
Tokyo 105-7325 (JP)**
• **NAKAMURA, Saeko
Tokyo 105-7325 (JP)**
• **SAKAGUCHI, Yasuyuki
Tokyo 105-7325 (JP)**

(74) Representative: **Strehl Schübel-Hopf & Partner
Maximilianstrasse 54
80538 München (DE)**

(54) **SOLID CATALYST, METHOD FOR PRODUCING SOLID CATALYST AND METHOD FOR PRODUCING MONOMETHYL AMINE**

(57) The present invention provides a solid catalyst which can suppress generation of trimethylamine, while ensuring a suitable conversion rate of a methylation agent, thereby efficiently producing monomethyl amine with high selectivity, when the solid catalyst is used in a reaction between ammonia and a methylation agent; and a method for producing the solid catalyst. The present invention provides a method for producing a solid catalyst that has a specific surface area of 4 to 60 m$^2$/g and that is a mordenite-type zeolite, the method comprising: a mixing step of mixing a raw material mordenite and a solution obtained by dissolving a basic salt in a solvent to form a mixed liquid; an evaporating-drying step of evaporating the mixed liquid to dryness to obtain a solution-treated mordenite; and a firing step of firing the solution-treated mordenite. It is preferable that a crystallinity of the solid catalyst is 80% or more.

EP 4 417 305 A1

Processed by Luminess, 75001 PARIS (FR)

**Description**

[Technical Field]

The present invention relates to a solid catalyst, a method for producing a solid catalyst, and a method for producing monomethylamine using the solid catalyst.

This application claims priority based on Japanese Patent Application No. 2021-166985 filed in Japan on October 11, 2021, the contents of which are incorporated herein.

[Background Art]

Methylamines are generally produced by reacting a methylating agent and ammonia in a gas phase at high temperatures (for example, around 400°C) in the presence of a solid acid catalyst with dehydration and amination effects such as alumina or silica alumina. This reaction produces monomethylamine, dimethylamine, and trimethylamine.

The demand of trimethylamine is significantly less compared to that of monomethylamine and dimethylamine. Therefore, the trimethylamine is transferred to the reaction system and reused. Furthermore, regarding monomethylamine and dimethylamine, the production amount often does not match the demand, and the components produced in excess are transferred to the reaction system and reused, similarly to trimethylamine.

A distillation method is used to separate each component of monomethylamine, dimethylamine, and trimethylamine from the product (mixture) obtained by the above reaction. However, the boiling points of these methylamines are close to each other. In particular, trimethylamine forms a complex azeotrope with ammonia, monomethylamine, and dimethylamine. Therefore, in order to separate each component from the product obtained by the above reaction, a complicated and large-scale distillation operation is required. Therefore, the energy consumption and cost in a collection process of methylamines are large.

A collection process for methylamines is described in, for example, Non-Patent Document 1.

Among methylamines, the demand for dimethylamine is particularly large. For this reason, techniques for producing dimethylamine have been developed.

For example, Patent Documents 1 to 3 disclose a method for producing dimethylamine by reacting ammonia and methanol in a gas phase in the presence of a mordenite catalyst containing sodium. Furthermore, Patent Document 3 discloses that the reaction rate decreases as the alkali metal content in the catalyst increases, and that when the alkali metal content exceeds a certain amount, the amount of monomethylamine produced increases and the production ratio of dimethylamine decreases.

Further, Patent Document 4 discloses a method for producing monomethylamine in which ammonia and methanol are reacted in the presence of a mordenite catalyst containing sodium.

Patent Document 5 discloses a method for producing methylamines via a gas phase catalytic reaction of ammonia and methanol. Furthermore, Patent Document 5 discloses that trimethylamine is distilled out as an azeotrope with ammonia, and that trimethylamine is azeotropically distilled with monomethylamine and dimethylamine.

[Citation List]

[Patent Documents]

[Patent Document 1] Japanese Patent No. 3995611
[Patent Document 2] Japanese Patent No. 4758645
[Patent Document 3] Japanese Patent Publication No. H02-16743
[Patent Document 4] Japanese Patent Publication No. S63-25575
[Patent Document 5] Japanese Patent Application Publication No. 2007-161637

[Non-Patent Documents]

[Non-Patent Document 1] "Complete collection of revised manufacturing process diagrams", pages 440-441, April 25, 1978, published by Kagaku Kogyosha Co., Ltd.

[Summary of the Invention]

[Problem to Be Solved by Invention]

Recently, the demand for N-methyl-2-pyrrolidone (NMP) has been increasing rapidly in fields such as a lithium ion battery production. NMP is a derivative of monomethylamine. For this reason, it has become important to establish a method for producing monomethylamine.

Monomethylamine can be produced by a method of reacting ammonia with a methylating agent such as methanol. However, in this method, monomethylamine produced by the reaction is likely to be converted into dimethylamine and trimethylamine for the reasons described below.

That is, monomethylamine has a structure in which one of the hydrogen atoms of ammonia is substituted with a methyl group. Monomethylamine is often more reactive with the methylating agent than ammonia because the methyl group is an electron-donating group. Therefore, monomethylamine is easily converted to dimethylamine by further reaction with the methylating agent. Furthermore, since dimethylamine has two methyl groups, it has higher reactivity with a methylating agent than monomethylamine. Therefore, it is more easily converted to trimethylamine by reaction of dimethylamine with the methylating agent.

Therefore, when monomethylamine is produced by reacting ammonia and a methylating agent, the production percentage of monomethylamine is increased due to an equilibrium theory, by reacting under conditions in which ammonia is present in excess with respect to stoichiometry.

Furthermore, trimethylamine, which is produced as a by-product from the reaction between ammonia and the methylating agent, is a component that is azeotropic with monomethylamine and therefore, it is difficult to separate the trimethylamine from monomethylamine. Therefore, there is a need for a method for producing monomethylamine which can increase the selectivity of monomethylamine by suppressing the production of trimethylamine.

Examples of the method for producing monomethylamine include a method in which a solid acid catalyst which has been partially neutralized by a base is used, and by shortening the reaction time between ammonia and the methylating agent, the reaction between dimethylamine and the methylating agent and the reaction between monomethylamine and the methylating agent were suppressed. By using the method, the reaction rate between a methylating agent and any compounds selected from ammonia, monomethylamine, dimethylamine, and trimethylamine can be reduced, and the reaction rate between two compounds selected from these compounds can also be reduced, and as a result, the selectivity of monomethylamine can be increased.

However, in a conventional method for producing monomethylamine, the production of trimethylamine could not be sufficiently suppressed, and the selectivity of monomethylamine could not be sufficiently increased. Furthermore, in order to increase the selectivity of monomethylamine, the reaction time between ammonia and the methylating agent must be shortened, and as a result, it is difficult to improve productivity.

The present invention was made in view of the above circumstances, and an object of the present invention is to provide a solid catalyst and a method for producing the same, wherein the solid catalyst can suppress the production of trimethylamine while ensuring the conversion rate of the methylating agent, and can efficiently produce monomethylamine at a high rate, when the reaction between ammonia and a methylating agent are carried out in the presence of the solid catalyst.

Furthermore, another object of the present invention is to provide a method for producing monomethylamine that can sufficiently suppress the production of trimethylamine even if the reaction time is increased, and that can efficiently produce monomethylamine with high selectivity while ensuring the conversion rate of the methylating agent.

[Means to Solve the Problem]

The present inventors have conducted extensive research to solve the above problems.

As a result, the present inventors have discovered that by reacting ammonia with a methylating agent in the presence of a solid catalyst of zeolite which has a mordenite crystal structure and has a specific surface area of 4 to 60 $m^2/g$ which is calculated by a BET method from the gas adsorption characteristics using nitrogen molecules as a probe, monomethylamine can be efficiently produced with high selectivity, by suppressing the production of trimethylamine sufficiently while ensuring the conversion rate of the methylating agent; and the present invention has been conceived.

That is, the present invention relates to the following matters.

[1] A method for producing a solid catalyst which has a specific surface area of 4 to 60 $m^2/g$ and is a mordenite-type zeolite, the method comprising:

a mixing step of mixing a raw material mordenite and a solution obtained by dissolving a basic salt in a solvent to form a mixed liquid;

an evaporating-drying step of evaporating the mixed liquid to dryness to obtain a solution-treated mordenite; and
a firing step of firing the solution-treated mordenite.

[2] The method for producing a solid catalyst according to [1], wherein the solid catalyst has a crystallinity of 80% or more.

[3] The method for producing a solid catalyst according to [1] or [2], wherein the solid catalyst is a solid catalyst for producing monomethylamine via a reaction between ammonia and a methylating agent.

[4] The method for producing a solid catalyst according to any one of [1] to [3],
wherein the firing step comprises

a first firing step of obtaining fired mordenite by firing the solution-treated mordenite;
a cleaning step of cleaning the fired mordenite with the solvent; and
a second firing step of firing the cleaned fired mordenite.

[5] The method for producing a solid catalyst according to any one of [1] to [4], wherein the basic salt contains a sodium ion as a cation.

[6] The method for producing a solid catalyst according to any one of [1] to [5], wherein the basic salt contains at least one selected from the group consisting of hydroxide ions and carbonate ions as anions.

[7] The method for producing a solid catalyst according to any one of [1] to [6], wherein the basic salt contains sodium hydroxide.

[8] A method for producing a solid catalyst which has a specific surface area of 4 to 60 $m^2/g$ and is a mordenite-type zeolite, the method comprising:

a mixing step of mixing a raw material mordenite and a solution obtained by dissolving a basic salt in a solvent to form a mixed liquid;
a stirring step of stirring the mixed liquid to obtain a solution-treated mordenite; and
a firing step of firing the solution-treated mordenite.

[9] The method for producing a solid catalyst according to [8], wherein the solid catalyst has a crystallinity of 80% or more.

[10] The method for producing a solid catalyst according to [8] or [9], wherein the solid catalyst is a solid catalyst for producing monomethylamine via a reaction between ammonia and a methylating agent.

[11] The method for producing a solid catalyst according to any one of [8] to [10], wherein the basic salt contains a sodium ion as a cation.

[12] The method for producing a solid catalyst according to any one of [8] to [11], wherein the basic salt contains a hydroxide ion as an anion.

[13] The method for producing a solid catalyst according to any one of [8] to [12], wherein the basic salt contains sodium hydroxide.

[14] A method for producing a solid catalyst which has a specific surface area of 4 to 60 $m^2/g$ and is a mordenite-type zeolite, the method comprising:
a firing step of firing a raw material mordenite at a temperature of 650°C to 1000°C.

[15] The method for producing a solid catalyst according to [14], wherein the solid catalyst has a crystallinity of 80% or more.

[16] The method for producing a solid catalyst according to [14] or [15], wherein the solid catalyst is a solid catalyst for producing monomethylamine via a reaction between ammonia and a methylating agent.

[17] A solid catalyst which has a specific surface area of 4 to 60 $m^2/g$ and is a mordenite-type zeolite, wherein the solid catalyst is produced by the method for producing a solid catalyst according to any one of [1] to [16].

[18] A method for producing monomethylamine, the method comprising:
reacting ammonia and a methylating agent in the presence of the solid catalyst according to [17].

[19] The method for producing monomethylamine according to [18], wherein the methylating agent is methanol.

[20] The method for producing monomethylamine according to [18] or [19], wherein a molar ratio of an amount of ammonia used and an amount of the methylating agent used is within a range of 0.2 to 5.

[21] The method for producing monomethylamine according to any one of [18] to [20], wherein a weight hourly space velocity in the reaction is within a range of 0.1 to 3.0 per hour.

[Advantageous Effects of Invention]

By using the solid catalyst of the present invention in the reaction of ammonia and a methylating agent for producing monomethylamine, the production of trimethylamine can be suppressed while ensuring the conversion rate of the methylating agent, and monomethylamine can be efficiently produced with high selectivity.

According to the method for producing monomethylamine of the present invention, the production of trimethylamine can be sufficiently suppressed even if the reaction time increases, and monomethylamine can be efficiently produced with high selectivity while ensuring the conversion rate of the methylating agent.

[Description of Embodiments]

In order to solve the above problems, the present inventors focused on the reaction mechanism between ammonia and the methylating agent and a pore dimension (pore size) of the solid catalyst used for the reaction, and did the following efforts.

Firstly, the reaction mechanism between ammonia and a methylating agent will be explained. Here, in order to specifically describe the reaction between ammonia and a methylating agent and the reaction product produced by the reaction, an example in which methanol is used as the methylating agent will be described.

Normally, when ammonia and methanol are reacted in the presence of an appropriate catalyst, it is assumed that at least the following (first reaction) to (fourth reaction) and their reverse reactions occur.

(First reaction) Ammonia and methanol react to produce monomethylamine ($CH_3NH_2$) and water ($H_2O$).

(Second reaction) Monomethylamine and methanol react to produce dimethylamine (($CH_3)_2NH$) and water ($H_2O$).

(Third reaction) Dimethylamine and methanol react to produce trimethylamine (($CH_3)_2NH$) and water ($H_2O$).

(Fourth reaction) Any two compounds selected from the four types of compounds: ammonia, monomethylamine, dimethylamine, and trimethylamine (they may be the same type of compounds or different types of compounds) react with each other. As a result, an exchange reaction proceeds between the hydrogen atom bonded to the nitrogen atom of one molecule and the methyl group bonded to the nitrogen atom of the other molecule.

In the four types of compounds involved in (first reaction) to (fourth reaction), that is, ammonia, monomethylamine, dimethylamine, and trimethylamine, the atom or atomic group bonded to the nitrogen atom is a hydrogen atom (-H) or a methyl group (-$CH_3$). Since the hydrogen atom is one of the atoms constituting the methyl group, which is an atomic group, the size of the methyl group is obviously larger than the size of the hydrogen atom. Therefore, based on the number of hydrogen atoms and methyl groups bonded to the nitrogen atom of each compound, it is obvious that the molecular sizes of these four types of compounds, when arranged in descending order, are ammonia < monomethylamine < dimethylamine < trimethylamine.

Based on this, the present inventors believe that in order to produce monomethylamine with high selectivity, it is necessary to use a solid catalyst for the reaction between ammonia and methanol, which can collect compounds having a size less than, for example, trimethylamine molecules, among the reaction products. In this case, the solid catalyst used only needs to be able to promote the reaction that produces monomethylamine, which is the target product, and the solid catalyst can be used regardless of whether it promotes the reaction that produces dimethylamine and the reaction that produces trimethylamine.

Therefore, as a raw material for a solid catalyst, the present inventors focused on mordenite, which can promote the above-mentioned (first reaction) to (fourth reaction), and conducted extensive studies. As a result, it was discovered that a solid catalyst which has a specific surface area of 4 to 60 $m^2$/g and has a crystal structure of mordenite-type zeolite and which is produced by any one of the methods (1) to (3) below can be used, and the present invention was conceived.

(1) A method including a mixing step in which a raw material mordenite and a solution obtained by dissolving a basic salt in a solvent are mixed to form a mixed liquid, an evaporating-drying step in which the mixed liquid is evaporated to dryness to obtain a solution-treated mordenite, and a firing step of firing the solution-treated mordenite.
(2) A method including a mixing step in which raw mordenite and a solution obtained by dissolving a basic salt in a solvent are mixed to form a mixed liquid, a stirring step in which the mixed liquid is stirred to form a solution-treated mordenite, a stirring step in which mordenite is obtained after solution treatment, and a firing step of firing the solution-treated mordenite.
(3) A method of firing a raw material mordenite at a temperature of 650 to 1000°C.

By reacting ammonia and methanol in the presence of the solid catalyst described above, the present inventors produced the monomethylamine with high selectivity and the amount of trimethylamine in the product was a trace amount. From this, it is presumed that the solid catalyst obtained by the above production method has micropores through which monomethylamine molecules can pass, but through which trimethylamine molecules cannot pass.

Hereinafter, the solid catalyst, the method for producing the solid catalyst, and the method for producing monomethylamine of the present invention will be explained in detail. Note that the present invention is not limited only to the embodiments shown below.

[Solid Catalyst]

The solid catalyst of the present embodiment has a specific surface area of 4 to 60 $m^2/g$ and is a mordenite-type zeolite.

Since the solid catalyst of the present embodiment is a zeolite with a mordenite crystal structure, it can effectively accelerate the reaction rate between ammonia and the methylating agent.

The specific surface area of the solid catalyst of the present embodiment is a numerical value determined by measurement using a BET method (surface area measurement method) using nitrogen molecules as probe molecules.

When the solid catalyst, which has a specific surface area of 60 $m^2/g$ or less and is mordenite-type zeolite, is produced by any one of the methods (1) to (3) above, it is presumed that in the solid catalyst, micropores are formed so that trimethylamine molecules cannot pass through. Therefore, when, for example, ammonia and a methylating agent are reacted in the presence of a solid catalyst with a specific surface area of 60 $m^2/g$ or less, only compounds smaller than the size of a trimethylamine molecule among the reaction products can be collected. Therefore, the production of trimethylamine can be suppressed and monomethylamine can be produced with high selectivity.

When the solid catalyst of the present embodiment is a solid catalyst used for the reaction between ammonia and a methylating agent, the specific surface area is preferably 50 $m^2/g$ or less, more preferably 30 $m^2/g$ or less, and even more preferably 25 $m^2/g$ or less.

The specific surface area of the solid catalyst of the present embodiment is 4 $m^2/g$ or more, preferably 5 $m^2/g$ or more, and more preferably 10 $m^2/g$ or more. For example, when ammonia and a methylating agent are reacted in the presence of a solid catalyst with a specific surface area of 4 $m^2/g$ or more, the reaction involving the methylating agent is promoted, and it can be ensured that the conversion rate of the methylating agent is sufficiently increased. As a result, monomethylamine can be produced efficiently.

The solid catalyst of the present embodiment preferably has a crystallinity of 80% or more. The crystallinity in the solid catalyst of the present embodiment is a value determined by powder X-ray diffraction analysis. The higher the crystallinity of the solid catalyst, the more the reaction involving the methylating agent can be promoted, and the higher the conversion rate of the methylating agent in the reaction between ammonia and the methylating agent can be increased. The crystallinity of the solid catalyst is more preferably 90% or more, even more preferably 95% or more, and may be 100%.

The solid catalyst of the present embodiment preferably contains sodium ions. Specifically, it is preferable that sodium ions are supported in the pores of the solid catalyst. For example, by reacting ammonia with a methylating agent in the presence of such a solid catalyst, monomethylamine can be produced with even higher selectivity.

The solid catalyst of the present embodiment has a specific surface area of 4 to 60 $m^2/g$ and is a mordenite-type zeolite. Therefore, the solid catalyst of the present embodiment is used in a reaction in which ammonia and a methylating agent are reacted to produce monomethylamine, thereby suppressing the production of trimethylamine while ensuring the conversion rate of the methylating agent, and monomethylamine can be produced efficiently with high selectivity.

[Method for Producing Solid Catalyst]

The solid catalyst of the present embodiment can be produced by using, for example, a first to a third production method shown below.

<First Production Method>

The first production method includes a mixing step in which a raw material mordenite and a solution obtained by dissolving a basic salt in a solvent are mixed to form a mixed liquid, an evaporating-drying step in which the mixed liquid is evaporated to dryness to form a solution-treated mordenite, and a firing step of firing the solution-treated mordenite.

(Mixing Step)

In the first production method, by performing a mixing step, the cations contained in the basic salt are ionically bonded to the acid sites of the raw material mordenite, and the cations are supported in the pores of the raw material mordenite, and at the same time, the solution obtained by dissolving the basic salt in a solvent is reacted with the raw material mordenite.

Examples of the raw material mordenite used in the mixing step include a mordenite-type zeolite, in which a molar ratio of $SiO_2$ to $Al_2O_3$ ($SiO_2/Al_2O_3$ ratio) is 10 to 250 and a specific surface area is 300 to 500 $m^2/gm$, and the like. The molar ratio of $SiO_2$ to $Al_2O_3$ in the raw material mordenite is preferably 10 to 50, and more preferably 12 to 20.

The raw material mordenite may be acidic mordenite or basic mordenite. As the raw material mordenite, it is preferable to use acidic mordenite because it can strongly adsorb basic ammonia and provide a solid catalyst that reacts more smoothly with the methylating agent.

As the raw material mordenite, commercially available products may be used. Specifically, acidic mordenite such as 620HOA (manufactured by Tosoh Corporation) and 640HOA (manufactured by Tosoh Corporation) can be used as the raw material mordenite.

The basic salt in the solution obtained by dissolving the basic salt in a solvent used in the mixing step is not particularly limited as long as it exhibits basicity when made into a solution. The basic salt preferably contains one or more alkali metal ions as cations, and more preferably contains $Na^+$ (sodium ions). The basic salt may contain cations such as metal cations other than $Na^+$ and metal-free ammonium ions. Further, the counter anion contained in the basic salt is preferably a conjugate base of a weak acid. The basic salt preferably contains at least one selected from hydroxide ions and carbonate ions as anions, and more preferably contains hydroxide ions to obtain a solid catalyst with high monomethylamine selectivity.

Examples of the basic salt include sodium hydroxide, potassium hydroxide, cesium hydroxide, sodium carbonate, sodium hydrogen carbonate, sodium phosphate, and the like. Among these, it is preferable to use sodium hydroxide as the basic salt because it can suppress the production of dimethylamine and provide a solid catalyst with higher monomethylamine selectivity.

The solvent for a solution obtained by dissolving the basic salt in a solvent may be any solvent without any limitation as long as that the solvent can dissolve the basic salt, can be removed relatively easily by evaporating-drying, and does not decompose when the solvent is being removed. Examples of the solvent include highly polar solvents such as water, methanol, ethanol, dimethylacetamide, dimethylsulfoxide, and hexamethylphosphoric triamide. Among these solvents, it is preferable to use one or more selected from water, methanol, and ethanol because they have a relatively low boiling point and can be easily removed, and it is more preferable to use water because of its low cost.

The concentration of the basic salt contained in the solution is preferably 0.01 mol/L or more, and more preferably 0.05 mol/L or more, because the amount of solvent in the solution is small and the time to evaporate the mixed liquid to dryness can be shortened.

Further, in order to obtain a uniform mixture of the raw material mordenite and the solution obtained by dissolving the basic salt in a solvent, the concentration of the basic salt contained in the solution is preferably 10 mol/L or less, more preferably 2.5 mol/L or less, and even more preferably 1.0 mol/L or less. When the concentration of the basic salt contained in the solution is 10 mol/L or less, the time required to evaporate the liquid mixture to dryness becomes sufficiently long. Further, in order to shorten the time for evaporating the mixed liquid to dryness, the heating temperature is increased when the mixed liquid is evaporated to dryness while being heated. In either case, if the concentration of the basic salt contained in the solution is 10 mol/L or less, the reaction between the raw material mordenite and the basic salt in the evaporating-drying step is promoted. Therefore, the surface of the raw material mordenite is sufficiently dissolved by the solution obtained by dissolving the basic salt in a solvent, and a reaction product between the basic salt and the raw material mordenite is sufficiently generated.

The amount of the basic salt used in the solution is appropriately decided depending on the ratio of the amount of the cations in the basic salt to the amount of the points which can provide cations in the raw material mordenite. The amount of points which can provide cations in the raw material mordenite can be determined, for example, by a method of measuring the amount of base adsorbed on the raw material mordenite, by a temperature programmed desorption (TPD) method using an appropriate base (for example, ammonia).

The ratio of the amount of cations in the basic salt contained in the solution to the amount of points in which cations can be provided in the raw material mordenite ((total of [valence $\times$ amount] of cations in the salt contained in the solution)/(amount of points in which cations can be provided in the raw material mordenite)) is preferably 0.5 or more, and more preferably 0.9 or more. When the ratio of the above amounts is 0.5 or more, the surface of the raw material mordenite is sufficiently dissolved by the solution obtained by dissolving the basic salt in a solvent, and the reaction product between the basic salt and the raw material mordenite which is sufficiently dissolved can generated. In addition, from the viewpoint of cost, the ratio of the amount of cations in the basic salt contained in the solution to the amount of points in which cations can be provided in the raw material mordenite is preferably 5 or less, and is more preferably 2.5 or less. Further, when the ratio of the amounts is 5 or less, it is possible to prevent the surface of the raw material mordenite from being excessively dissolved by the solution obtained by dissolving the basic salt in a solvent.

The method of mixing the raw material mordenite and the solution obtained by dissolving the basic salt in a solvent to form a mixed liquid is not particularly limited. For example, a method of immersing the raw material mordenite in the solution obtained by dissolving the basic salt in a solvent can be used.

(Evaporating-Drying Step)

In the evaporating-drying step, the liquid mixture of the raw material mordenite and the solution obtained by dissolving the basic salt in a solvent is evaporated to dryness to obtain a solution-treated mordenite.

The method of evaporating-drying can be appropriately determined depending on the type of solvent contained in the liquid mixture, and is not particularly limited. When the solvent is water, the reaction may be carried out, for example, by leaving it standing in an atmospheric environment at room temperature for an appropriate period of time. In this case, the time for standing still is not particularly limited as long as the solvent can be removed, and may be, for example, 5 to 14 days, 7 to 12 days, or about 10 days. The evaporating-drying step may be carried out by heating in an atmosphere for an appropriate period of time using an oven set at a temperature that is above room temperature and below the boiling point of the solvent and that does not cause bumping of the solvent. By using this method, the time required to complete evaporating-drying can be shortened. The time for heating using an oven may be adjusted as appropriate depending on the heating temperature. For example, when the solvent is water, the treatment may be carried out at a temperature of about 80°C for 5 to 10 hours, or may be carried out at a temperature of about 50°C for 40 to 60 hours.

(Firing Step)

In the firing step, the solution-treated mordenite obtained by performing the evaporating-drying step is fired. By performing the firing step, a component generated by the reaction between the basic salt and the raw material mordenite in the mixing step and the evaporating-drying step is precipitated and fixed on the surface of the raw material mordenite.

It is preferable that the firing step of the first production method includes a first firing step in which a fired mordenite is obtained by firing the solution-treated mordenite; a cleaning step in which the fired mordenite is cleaned with a solvent; and a second firing step in which the fired mordenite after cleaning is fired.

The solution-treated mordenite in the first firing step can be fired, for example, by heating at 300 to 600°C for 1 to 20 hours in an air atmosphere. The solution-treated mordenite is preferably fired by heating at 450 to 550°C for 6 to 15 hours in an air atmosphere. When the firing temperature in the first firing step is 600°C or lower, it is possible to prevent the crystal structure of the surface of the raw material mordenite from being destroyed by firing.

The solution-treated mordenite may or may not be cleaned before the first firing step, but it is preferable to perform the first firing step without cleaning. By performing the first firing step without cleaning the solution-treated mordenite, the component generated by the reaction between the basic salt and the raw material mordenite becomes more likely to be fixed on the surface of the raw material mordenite.

In the cleaning step, the fired mordenite obtained in the first firing step is cleaned with a solvent. As the solvent, a solvent used for the solution obtained by dissolving the basic salt in a solvent is used. By performing the cleaning step, solvent-soluble components such as excess salt remaining on the surface of the fired mordenite can be removed.

The method for cleaning the fired mordenite is not particularly limited, and examples of the methods include a method in which the fired mordenite is placed on an appropriate filter medium and a solvent is passed through it; a method in which a glass column is packed with the fired mordenite and a solvent is passed through it; and a method in which a solvent and the fired mordenite are placed in a beaker, are stirred, and are allowed to stand, and the supernatant liquid is then removed with a pipette.

The fired mordenite after cleaning in the second firing step can be fired in the same manner as in the first firing step, for example, by heating at 300 to 600°C for 1 to 20 hours in an air atmosphere. The fired mordenite is preferably fired by heating at 450 to 550°C for 6 to 15 hours in an air atmosphere.

The firing conditions in the second firing step may be the same as or different from the firing conditions in the first firing step.

Through the above steps, the solid catalyst of the present embodiment can be obtained.

The solid catalyst produced by the first production method may maintain the cations constituting the basic salt used in the production process. Therefore, if necessary, the produced solid catalyst may be treated with a strong acid such as hydrochloric acid, nitric acid, or sulfuric acid to replace the cations supported by the solid catalyst with protons.

The solid catalyst produced by the first production method is estimated to have a mordenite layer and a surface layer formed on the surface of the mordenite layer. The mordenite layer has a mordenite-type crystal structure and has pores derived from the raw material mordenite used as a raw material for the solid catalyst. The surface layer has micropores through which monomethylamine molecules can pass, but through which trimethylamine molecules cannot pass.

By performing the first production method in the present embodiment, it is estimated that the raw material mordenite changes as shown below and becomes the solid catalyst.

When the mixing step and the evaporating-drying step in the first production method are performed, the raw material mordenite reacts with the solution obtained by dissolving the basic salt in a solvent, and cations are supported on the raw mordenite, and at the same time, some components of the raw material mordenite are dissolved. Examples of the components to be dissolved include sodium oxide. At this time, the mordenite layer of the solid catalyst is formed from the raw material mordenite that remains undissolved, and the solid catalyst preferably has a crystallinity of 80% or more.

In addition, through the firing step of the first production method, the dissolved raw material mordenite components precipitate on the surface of the raw material mordenite that maintains crystallinity, and form a layer that no longer maintains the crystal structure of the mordenite. This layer is fixed on the surface of the raw material mordenite by the above-mentioned firing step, and becomes a surface layer having micropores. Monomethylamine molecules can pass through the micropores, but trimethylamine molecules cannot pass through the micropores.

It is estimated that the smaller the specific surface area is, the smaller the micropores of the solid catalyst produced by the first production method are, and it is presumed that it is not possible to allow trimethylamine molecules to pass through the micropores of the solid catalyst with a specific surface area of 60 $m^2$/g or less.

In the first production method, solid catalysts with different specific surface areas can be produced by changing the ratio of the amount of cations in the basic salt contained in the solution to the amount of points in which cations can be provided in the raw material mordenite ((total of [valence $\times$ amount] of cations in the salt contained in the solution)/(amount of points in which cations can be provided in the raw material mordenite)), and the concentration of the basic salt contained in the solution obtained by dissolving the basic salt in a solvent.

Therefore, the size of the micropores in the solid catalyst can be controlled by changing the ratio of the amount of cations in the basic salt contained in the solution to the amount of points in which cations can be provided in the raw material mordenite, and the concentration of the basic salt contained in the solution obtained by dissolving the basic salt in a solvent.

Moreover, the solid catalyst produced by the first production method tends to have a smaller specific surface area as the crystallinity becomes lower.

In addition, since the solid catalyst produced by the first production method is produced by a production method that includes an evaporating-drying step, in the solid catalyst, a surface layer is formed on a structure having a crystallinity of 80% or more, by not excessively dissolving the raw material mordenite via the solution obtained by dissolving the basic salt in a solvent, wherein the surface layer has micropores large enough to allow monomethylamine molecules to easily pass through.

The first production method of the present embodiment includes a mixing step of mixing the raw material mordenite and the solution obtained by dissolving the basic salt in a solvent to form a mixed liquid, an evaporating-drying step of evaporating the mixed liquid to dryness to form a solution-treated mordenite, and a firing step of firing the solution-treated mordenite. Therefore, the solid catalyst of the present embodiment, which has a specific surface area of 4 to 60 $m^2$/g and is a mordenite-type zeolite, can be obtained.

<Second Production Method>

The second production method includes a mixing step of mixing a raw material mordenite and a solution obtained by dissolving the basic salt in a solvent to form a mixed liquid; a stirring step of stirring the mixed liquid to obtain a solution-treated mordenite; and a firing step of firing the solution-treated mordenite.

(Mixing Step)

In the second production method, as in the first production method, by performing the mixing step, cations contained in the basic salt are ionically bonded to the acid sites of the raw material mordenite, and the cations are supported in the pores of the raw material mordenite, and at the same time, the solution obtained by dissolving a basic salt in a solvent is reacted with the raw material mordenite.

As the raw material mordenite and the solution obtained by dissolving the basic salt in a solvent, the same raw material mordenite and the same solution as used in the first production method can be used.

In the stirring step in the second production method, the concentration of the basic salt in the mixture does not change, unlike in the first production method in which the concentration of the basic salt in the mixture increases in the evaporating-drying step. For this reason, in order to ensure that the reaction between the basic salt in the mixture and the raw material mordenite proceeds sufficiently in the stirring step, it is preferable to use a strongly basic salt such as sodium hydroxide as the basic salt.

The concentration of the basic salt contained in the solution is preferably 0.1 mol/L or more, and more preferably 0.2 mol/L or more in order to allow the reaction between the basic salt and the raw material mordenite to proceed sufficiently.

Further, the concentration of the basic salt contained in the solution is preferably 10 mol/L or less, and more preferably 5 mol/L or less. When the concentration of the basic salt contained in the solution is 10 mol/L or less, the reaction between the raw material mordenite and the basic salt does not proceed excessively. Therefore, the mordenite-type crystal structure that contributes to the promotion of the monomethylamine production reaction is not dissolved in excess, and a solid catalyst that can ensure a sufficient conversion rate of the methylating agent can be obtained.

The ratio of the amount of cations in the basic salt contained in the solution to the amount of points in which cations can be provided in the raw material mordenite ((total of [valence × amount] of cations in the salt contained in the solution)/(amount of points in which cations can be provided in the raw material mordenite)) is preferably 10 or more, and more preferably 15 or more. When the ratio of the amounts of the above substances is 10 or more, the surface of the raw material mordenite is sufficiently dissolved by the solution obtained by dissolving the basic salt in a solvent, and the reaction product between the basic salt and the raw material mordenite is sufficiently generated. Further, from the viewpoint of cost, the ratio of the amount of cations in the basic salt contained in the solution to the amount of points in which cations can be provided in the raw material mordenite is preferably 75 or less, and is more preferably 50 or less. Further, when the ratio of the amounts of substances is 75 or less, it is possible to prevent the surface of the raw material mordenite from being excessively dissolved by a solution obtained by dissolving the basic salt in a solvent.

As a method for mixing the raw material mordenite and the solution obtained by dissolving the basic salt in a solvent to form a mixed liquid, for example, a method similar to the method used in the first production method can be used.

(Stirring Step)

In the stirring step, a mixed liquid of the raw material mordenite and the solution obtained by dissolving the basic salt in a solvent is stirred to obtain a solution-treated mordenite. A known method can be used to stir the liquid mixture, and there is no particular limitation.

The stirring time for the mixed liquid can be determined as appropriate depending on the concentration of the basic salt contained in the solution, and, for example, at room temperature in the air, the stirring time may be 0.5 to 48 hours. When the stirring time is 0.5 hours or more, the surface of the raw material mordenite is sufficiently dissolved by the solution obtained by dissolving the basic salt in a solvent, and a reaction product between the basic salt and the raw material mordenite is sufficiently generated. When the stirring time is 48 hours or less, it is possible to prevent the surface of the raw material mordenite from being excessively dissolved by the solution obtained by dissolving the basic salt in a solvent. The stirring time is more preferably 1 to 24 hours, and even more preferably 6 to 15 hours.

In the second production method, it is preferable that the solution-treated mordenite obtained by performing the stirring step is filtered and cleaned, for example, by a natural filtration method, a suction filtration method, or the like.

As the method of cleaning the solution-treated mordenite, for example, a method of passing the solvent used in the solution obtained by dissolving the basic salt in the solvent, through the solution-treated mordenite collected by filtration may be used.

(Firing Step)

In the firing step, the solution-treated mordenite obtained by performing the stirring step is fired. By performing the firing step, the component produced by the reaction between the basic salt and the raw material mordenite in the mixing step and the stirring step is precipitated and fixed on the surface of the raw material mordenite.

Similarly to firing the solution-treated mordenite in the first production method, firing the solution-treated mordenite in the firing step of the second production method, for example, may be carried out by a heating method at 300 to 600°C for 1 to 20 hours in an air atmosphere. It is preferably carried out at 450 to 550°C for 6 to 15 hours in an air atmosphere.

By carrying out the above steps, the solid catalyst of the present embodiment can be obtained.

The solid catalyst produced by the second production method may maintain the cations constituting the basic salt used in the production process, similarly to the solid catalyst produced by the first production method. Therefore, if necessary, the produced solid catalyst may be treated with a strong acid such as hydrochloric acid, nitric acid, or sulfuric acid to replace the cations supported by the solid catalyst with protons.

The solid catalyst produced by the second production method is estimated to have a mordenite layer and a surface layer formed on the surface of the mordenite layer, similarly to the solid catalyst produced by the first production method. The mordenite layer has a mordenite-type crystal structure and has micropores derived from the raw material mordenite used as a raw material for the solid catalyst. The surface layer has the micropores through which monomethylamine molecules can pass, but through which trimethylamine molecules cannot pass.

By performing the second production method in the present embodiment, it is estimated that the raw material mordenite changes as shown below and become a solid catalyst.

That is, similarly to the case where the mixing step and the evaporating-drying step in the first production method are performed, when the mixing step and the stirring step in the second production method are performed, the reaction between the raw material mordenite and the solution obtained by dissolving the basic salt in a solvent is carried out, and as a result, cations are supported on the raw material mordenite, and at the same time, some components of the raw material mordenite are dissolved.

At this time, the mordenite layer of the solid catalyst is formed from the raw material mordenite that remains undissolved.

Similarly to the firing step in the first production method, in the firing step in the second production method, the dissolved raw material mordenite components precipitate on the surface of the raw material mordenite that maintains crystallinity, and form a surface layer with micropores. In the second production method, the stirring step is performed instead of the evaporating-drying step in the first production method, and therefore, less raw material mordenite is dissolved than that in the first production method, and as a result, the thickness of the surface layer is very thin. A solid catalyst is formed with a crystallinity close to 100%.

The size of the micropores can be controlled by changing the conditions of the mixing step and the stirring step in the second production method.

Similarly to the solid catalyst produced by the first production method, it is estimated that the smaller the micropores of the solid catalyst produced by the second production method are, the smaller the specific surface area of the solid catalyst is, and it is presumed that trimethylamine molecules cannot pass through the micropores of the solid catalyst with the specific surface area of 60 $m^2/g$ or less.

In the second production method, solid catalysts with different specific surface areas can be produced by changing one or more conditions selected from the ratio of the amount of cations in the basic salt contained in the solution to the amount of points in which cations can be provided in the raw material mordenite ((total of [valence $\times$ amount] of cations in the salt contained in the solution)/(amount of points in which cations can be provided in the raw material mordenite)), the concentration of the basic salt contained in the solution obtained by dissolving the basic salt in a solvent, and the stirring time of the mixed liquid.

Therefore, the size of the micropores in the solid catalyst can be controlled by changing one or more conditions selected from the ratio of the amount of cations in the basic salt contained in the solution to the amount of points in which cations can be provided in the raw material, the concentration of the basic salt contained in the solution obtained by dissolving the basic salt in a solvent, and the stirring time of the mixed liquid.

The second production method of the present embodiment includes a mixing step of mixing the raw material mordenite and the solution obtained by dissolving the basic salt in a solvent to form a mixed liquid; a stirring step of stirring the mixed liquid to form a solution-treated mordenite; and a firing step of firing the solution-treated mordenite. Therefore, the solid catalyst of the present embodiment, which has a specific surface area of 4 to 60 $m^2/g$ and is a mordenite-type zeolite, can be obtained.

<Third Production Method>

The third production method includes a firing step (high-temperature firing step) in which a raw material mordenite is fired at a temperature of 650 to 1000°C.

As the raw material mordenite, commercially available products may be used. Specifically, basic mordenite 642NAA (manufactured by Tosoh Corporation) can be used as the raw material mordenite.

In the high-temperature firing step, the raw material mordenite is fired at a temperature of 650 to 1000°C. As a result, the pores of the mordenite structure of the raw material mordenite are partially or uniformly destroyed. As a result, the specific surface area becomes 60 $m^2/g$ or less, and it is estimated that micropores are formed through which monomethylamine molecules can pass, but through which trimethylamine molecules cannot pass.

Therefore, unlike the solid catalysts produced by the first production method and the second production method, the solid catalyst produced by the third production method may not have a surface layer formed on the surface of the mordenite layer, and micropores may not be formed on the surface layer.

Since the firing temperature in the high-temperature firing step is 650°C or higher, the mordenite structure of the raw material mordenite is destroyed, and a solid catalyst having a specific surface area of 60 $m^2/g$ or less is obtained. The firing temperature is preferably 700°C or higher. Furthermore, since the firing temperature is 1000°C or lower, the mordenite structure of the raw material mordenite may not be deactivated due to being destroyed by performing the high-temperature firing step. Therefore, when the reaction between ammonia and a methylating agent is carried out, the solid catalyst can ensure a sufficient conversion rate of the methylating agent. The firing temperature is preferably 800°C or less.

The firing time in the high-temperature firing step may be appropriately adjusted depending on the firing temperature within a range such that the mordenite structure of the raw material mordenite is not deactivated due to being destroyed, and the firing time may be, for example, 0.5 to 2 hours.

The third production method of the present embodiment includes a high-temperature firing step of firing the raw material mordenite at a temperature of 650 to 1000°C. Therefore, the solid catalyst of the present embodiment, which has a specific surface area of 4 to 60 $m^2/g$ and is a mordenite-type zeolite, can be obtained.

[Method for Producing Monomethylamine]

Next, the method for producing monomethylamine of the present embodiment will be described in detail.

In the method for producing monomethylamine of the present embodiment, ammonia and a methylating agent are reacted in a gas phase in the presence of the solid catalyst of the present embodiment described above.

Examples of the methylating agent include methanol, dimethyl ether, chloromethane, bromomethane, iodomethane, and the like. Among these, methanol is preferably used as the methylating agent because it is inexpensive, liquid at room temperature, easy to handle, and relatively less harmful.

The raw material molar ratio, which is the molar ratio between the amount of ammonia used and the amount of the methylating agent used (ammonia/methylating agent), is preferably within a range of 0.2 to 5, more preferably 0.25 to 4, and even more preferably 0.33 to 3. If the raw material molar ratio (ammonia/methylating agent) is close to 1.0, which is the stoichiometric amount when monomethylamine is the target product, it will be advantageous in terms of cost because the excess or deficiency of raw materials will be small. On the other hand, when the raw material molar ratio deviates from 1.0, the conversion rate of the component, which is insufficient from the stoichiometric amount among the raw materials, increases. For example, when the raw material molar ratio is greater than 1.0, the conversion rate of the methylating agent tends to increase as the raw material molar ratio increases, since the stoichiometric amount of the methylating agent decreases. Therefore, when the raw material molar ratio deviates from 1.0, the unreacted amount of the above-mentioned components, in which the stoichiometric amount is insufficient in the reaction product, decreases. As a result, purification of the reaction product becomes easy, when it is difficult to remove the above-mentioned components from the target product. Moreover, when the raw material molar ratio is small, the probability of collision between methylating agents increases. As a result, the conversion rate of the methylating agent increases, and by-products generated by the reaction of the methylating agents tend to be generated more easily. From these viewpoints, the molar ratio of raw materials (ammonia/methylating agent) is preferably within the range of 0.2 to 5.

The temperature of the reaction between ammonia and the methylating agent may be within the range in which the above-mentioned (first reaction) and (second reaction) proceed, and it is preferable that the temperature is within a range in which the above-mentioned (fourth reaction) and the reverse reactions of the above-mentioned (first reaction) and (second reaction) proceed. Since the reaction rate decreases as the reaction temperature decreases, the lower limit of the reaction temperature is preferably 200°C or higher, more preferably 250°C or higher, even more preferably 300°C or higher, and still more preferably 350°C or higher. In order to improve the safety of the synthesis reaction and the durability of the reaction vessel, the upper limit of the reaction temperature is preferably 600°C or less, more preferably 500°C or less, even more preferably 450°C or less, and still more preferably 400°C or less.

In the reaction between ammonia and a methylating agent, a weight hourly space velocity (WHSV), which represents the ratio of the weight of the raw material mixture to the weight of the catalyst per unit time, is preferably within a range of 0.1 to 3.0 per hour, more preferably within a range of 0.2 to 2.0 per hour, and even more preferably within a range of 0.25 to 1.5 per hour. By controlling the weight space velocity per unit time to 3.0 per hour or less, the raw material can be sufficiently converted. Further, when the weight hourly space velocity per unit time is 0.1 per hour or more, it is not necessary to use a large amount of a solid catalyst or it is not necessary to use a device that stably maintains a low flow rate of the raw material mixture, which is preferable.

In the method for producing monomethylamine of the present embodiment, the reaction between ammonia and a methylating agent are carried out in the presence of the solid catalyst of the present embodiment, which has a specific surface area of 4 to 60 $m^2/g$ and is a mordenite-type zeolite. Therefore, according to the method for producing monomethylamine of the present invention, the production of trimethylamine can be sufficiently suppressed even if the reaction time is increased, and monomethylamine can be efficiently produced with high selectivity while ensuring the conversion rate of the methylating agent.

More specifically, on the surface of the solid catalyst of the present embodiment, micropores are formed, wherein monomethylamine molecules can pass through the micropores but trimethylamine molecules cannot pass through the micropores. Therefore, it is presumed that the reaction between ammonia and the methylating agent can be controlled as shown in (1) to (5) below, regardless of the length of time.

(1) When ammonia and the methylating agent pass through micropores and come into contact with reaction points existing in the pores originating from the raw material mordenite used as the raw material for the solid catalyst, the reaction between ammonia and the methylating agent occurs. The reaction is accelerated, and ammonia and the methylating agent react (see (first reaction) to (fourth reaction) described above).

(2) Ammonia, monomethylamine, dimethylamine, and trimethylamine are generated within the pores originating from the raw material mordenite.

(3) Among the ammonia, monomethylamine, dimethylamine, and trimethylamine which are produced, ammonia, monomethylamine, and dimethylamine that can pass through the micropores of the solid catalyst are desorbed from the solid catalyst and collected as reaction products. If the micropores of the solid catalyst are such that dimethylamine molecules cannot pass through, ammonia and monomethylamine are desorbed from the solid catalyst and collected as reaction products.

(4) Trimethylamine that cannot pass through the micropores of the solid catalyst (if the micropores are such that dimethylamine molecules cannot pass through, trimethylamine and dimethylamine) remains in the micropores originating from the raw material mordenite of the solid catalyst.

(5) Some of the trimethylamines (trimethylamine and dimethylamine if the micropores are such that dimethylamine molecules cannot pass through) remaining in the micropores derived from the raw material mordenite for the solid catalyst perform a reaction (for example, the reverse reactions of (first reaction) to (fourth reaction) described above, or (fourth reaction)) in the micropores and are converted into molecules having a small size that can pass through the micropores of the solid catalyst. Then, the molecules pass through the micropores, desorb from the solid catalyst, and are collected as a reaction product.

Therefore, in the method for producing monomethylamine of the present embodiment, trimethylamine (if the micropores are such that dimethylamine molecules cannot pass through, trimethylamine and dimethylamine) may be produced, but the trimethylamine cannot be collected as a product. That is, in the method for producing monomethylamine of the present embodiment, the yield of trimethylamine (trimethylamine and dimethylamine if the micropores are such that dimethylamine molecules cannot pass through) does not increase even if the reaction time is increased, and as a result, the selectivity of monomethylamine is not affected. Therefore, in the method for producing monomethylamine of the present embodiment, productivity can be improved by lengthening the reaction time, and monomethylamine can be produced with high selectivity.

In the method for producing monomethylamine of the present embodiment, when a solid catalyst having micropores through which dimethylamine molecules can pass is used, a mixture of monomethylamine and dimethylamine is produced. Monomethylamine and dimethylamine can be separated, for example, by a distillation method.

<Application Examples of Monomethylamine>

Monomethylamine can be suitably used for the synthesis of monomethylamine derivatives. Examples of such derivatives include N-methyl-2-pyrrolidone (NMP).

As a method for producing NMP, for example, a method of mixing monomethylamine with gamma-butyrolactone, and heating the mixture can be used.

[EXAMPLES]

Hereinafter, the present invention will be explained in more detail with reference to Examples and Comparative Examples. Note that the present invention is not limited to the following examples.

(Example 1)

"Manufacture of Solid Catalyst"

A solid catalyst was produced using the first production method.

That is, 1.3 g of sodium hydroxide (NaOH, manufactured by Junsei Kagaku Co., Ltd.) was dissolved in pure water, the volume was adjusted to 100 mL using a volumetric flask, and a solution obtained by dissolving the basic salt in a solvent (0.33 mol/L) was prepared. In a glass container, 15 g of acidic mordenite (trade name: 620HOA, manufactured by Tosoh Corporation) having $H^+$ as a cation which is a raw material mordenite, and the entire amount of the above-mentioned aqueous solution were mixed (mixing step).

In addition, the amount of points in which cations can be provided was 2.0 mmol per 1 g of the raw material mordenite. In Example 1, the ratio of the amount of cations in the basic salt contained in the solution to the amount of points in which cations can be provided was 1.1.

Thereafter, the solution was left to stand in an oven set at 80°C under an air atmosphere for 8 hours, and was evaporated to dryness to obtain a solution-treated mordenite (evaporating-drying step). After the resulting solution-treated mordenite was allowed to cool, it was transferred to a magnetic crucible and fired in an electric furnace set at 500°C in an air atmosphere for 12 hours to obtain a fired mordenite (first firing step).

The fired mordenite after being left to cool was transferred to a beaker, 100 mL of pure water was added, and after being stirred for 1 hour, it was allowed to stand, and the supernatant liquid was removed with a pipette, and the above-mentioned operation was repeated three times for cleaning (cleaning step). The cleaned fired mordenite was transferred to a magnetic crucible again and was dry-fired in an electric furnace set at 500°C in an air atmosphere for 12 hours (second firing step) to obtain the solid catalyst of Example 1.

The specific surface area and the crystallinity of the obtained solid catalyst of Example 1 were determined by the methods shown below. As a result, as shown in Table 1, the specific surface area of the solid catalyst of Example 1 was 15.8 m$^2$/g, and the crystallinity was 98.3%.

[Specific Surface Area]

The specific surface area was determined by measuring the gas adsorption properties of the solid catalyst using a specific surface area measuring device (BELSORP-mini II, manufactured by Microtrac Bell Co., Ltd.) at liquid nitrogen temperature using nitrogen molecules as a probe, and analyzing using the BET method.

[Crystallinity]

The crystallinity was calculated as follows using HighScore, which is a software manufactured by Malvern Panalytical Ltd, based on the profile of X-ray diffraction measurement measured by the method described below.

(1) A peak search was performed on the profile.
(2) A peak representing an amorphous state was inserted at the position of 2θ=24° with a half width of 12° and a total integrated intensity of 1000 cts×°2θ.
(3) By fixing the peak position, profile fitting was performed on the peak intensity, half-width, and shape parameters.
(4) The crystallinity (%) was calculated using the following formula from the total of the integrated intensities of each peak obtained and the integrated intensity of the peak at the 24° position.

[Math 1]

$$\text{Crystallinity} = \frac{I_{total} - I_{amorphous}}{I_{total}} \times 100$$

$I_{total}$: Total Integrated Intensity
$I_{amorphous}$: Integrated Intensity of Amorphous

<X-ray Diffraction Measurement>

X-ray diffraction measurements were carried out under the following conditions, by packing the solid catalyst in a glass holder with a depth of 0.5 mm.

- Equipment: MPD X'Pert Pro manufactured by Panalytical Ltd.

- X-ray source: Cu Kα ray

- Incoming X-ray side filter: 10 mm brass mask

- Detector side filter: Ni filter

- Incidence side slit: Soller slit 0.04 rad ASS 1/8°

- Detector side slit: ASS 5.0 mm Soller slit 0.04 rad

- Detector: PIXcel$^{1D}$

- Measurement method: Reflection method

- Operation range: 2θ=5° to 120° range

- Step width: 0.006565°

- Counting time: 78.795 sec/step

"Manufacture of Monomethylamine"

Next, in the presence of the solid catalyst of Example 1, ammonia and a methylating agent were reacted to produce monomethylamine by the method shown below.

That is, using a pellet die set with an exhaust port (GS3300, manufactured by Specac Ltd.), 2.0 g of the solid catalyst was pressurized at 4 tons for 15 minutes using a manual hydraulic press (manufactured by Specac Ltd.) to form catalyst tablets. The obtained catalyst tablets were crushed to obtain catalyst particles that passed through a 0.9 mm mesh sieve but did not pass through a 0.4 mm mesh sieve. 1.0 g of these catalyst particles were packed into a reaction tube.

Ammonia gas was supplied from a cylinder to the reaction tube with a primary pressure set at 0.5 MPa. Methanol as the methylating agent was supplied from a 10 mL gas-tight syringe to the reaction tube using a syringe pump (manufactured by As One Corporation, SPS-1). The reaction tube was heated using a mantle heater (manufactured by Tokyo Glass Equipment Co., Ltd., P2-5).

The reaction temperature was 390°C. Further, the raw material molar ratio, which is the molar ratio of ammonia to methanol (ammonia/methanol), was set to 3.0. The ammonia flow rate was 0.50 L/hour, and the methanol flow rate was 0.27 mL/hour. Ammonia and methanol were fed into the reaction tube together with a small amount of nitrogen gas to prevent backflow of ammonia, and the reaction was carried out at a weight hourly space velocity (WHSV) of 0.55 per hour.

An aqueous solution containing the reaction product was obtained by connecting the outlet of the reaction tube to a collection bottle which was filled with 50 mL of pure water and cooled with ice from the outside.

The reaction continued until the feed amount of ammonia and methanol was 0.64 g and 0.50 g, respectively.

The composition of the reaction product obtained by the reaction of Example 1 was analyzed using gas chromatography (GC).

As a result, monomethylamine, which is the target product, was detected from the reaction product; and as by-products, dimethylamine, trimethylamine, dimethyl ether, and unreacted raw materials, ammonia and methanol, were also detected.

The conversion rate of methanol (MeOH), selectivity of monomethylamine ($MeNH_2$), and each yield of monomethylamine, dimethylamine ($Me_2NH$), and trimethylamine ($Me_3N$) in the reaction of Example 1 were determined by the methods shown below. The results are shown in Table 1.

(Calculation of Methanol Conversion Rate and Yield/Selectivity of Alkylamines)

A calibration curve for each compound was determined by the method shown below.

Each of methanol (manufactured by Fujifilm Wako Pure Chemical Industries, Ltd., super dehydration, for organic synthesis), methylamine, dimethylamine, and trimethylamine (all manufactured by Tokyo Chemical Industry Co., Ltd., aqueous solutions) were diluted, respectively, and three or more samples with different concentrations were prepared. Each sample was analyzed using gas chromatography (GC), and a calibration curve was determined from the obtained peak areas.

Next, using the calibration curve determined by the above method, the yield and the conversion rate were calculated by the method shown below.

The aqueous solution containing the reaction product was analyzed using gas chromatography (GC), and the peak areas of methylamine, dimethylamine, and trimethylamine were determined. Then, based on the calibration curve obtained by the above method and the mass of the aqueous solution containing the reaction product, each content of methanol, methylamine, dimethylamine, and trimethylamine in the aqueous solution containing the reaction product was calculated, and moles of each sample were determined.

Then, the conversion rate (%) of methanol was determined by subtracting 1 from the ratio of the moles of unreacted methanol in the aqueous solution containing the reaction product with respect to the moles of methanol used as a raw material.

Furthermore, the yield of monomethylamine was determined by calculating the ratio of the moles of monomethylamine in the aqueous solution containing the reaction product with respect to the moles of methanol used as a raw material.

Furthermore, the selectivity of monomethylamine was determined by calculating the ratio of the yield of monomethylamine with respect to the conversion rate of methanol.

In addition, the yield of dimethylamine was determined by calculating the ratio of moles of dimethylamine in the aqueous solution containing the reaction product with respect to the moles of methanol used as a raw material.

Further, the yield of trimethylamine was determined by calculating the ratio of the moles of trimethylamine in the aqueous solution containing the reaction product to the moles of methanol used as a raw material.

[Table 1]

| | BET [m²/g] | Crystallinity [%] | Raw material molar ratio | WHSV [h⁻¹] | MeOH conversion rate [%] | MeNH₂ selectivity [%] | MeNH₂ yield [%] | Me₂NH yield [%] | Me₃N yield [%] |
|---|---|---|---|---|---|---|---|---|---|
| Example 1 | 15.8 | 98.3 | 3.0 | 0.55 | 85 | 95 | 81 | trace amount | trace amount |
| Example 2 | 15.8 | 98.3 | 3.0 | 1.1 | 68 | 92 | 62 | trace amount | trace amount |
| Example 3 | 15.8 | 98.3 | 1.0 | 0.98 | 72 | 87 | 63 | trace amount | trace amount |
| Example 4 | 15.8 | 98.3 | 0.5 | 1.6 | 33 | 93 | 31 | trace amount | trace amount |
| Example 5 | 12.5 | 94.8 | 3.0 | 0.55 | 67 | 91 | 61 | trace amount | trace amount |
| Example 6 | 16.5 | 95.6 | 3.0 | 0.55 | 63 | 96 | 61 | trace amount | trace amount |
| Example 7 | 6.2 | 93.5 | 3.0 | 0.55 | 60 | 78 | 46 | 2.4 | trace amount |
| Example 8 | 17.0 | 100 | 3.0 | 0.55 | 91 | 96 | 87 | trace amount | trace amount |
| Example 9 | 53.8 | none | 3.0 | 0.55 | 85 | 78 | 66 | 7.5 | trace amount |
| Example 10 | 27.0 | none | 3.0 | 0.55 | 71 | 89 | 63 | 1.2 | trace amount |
| Example 11 | 32.4 | none | 3.0 | 0.55 | 76 | 97 | 74 | 2.3 | trace amount |
| Example 12 | 9.37 | none | 3.0 | 0.55 | 62 | 87 | 54 | 2.9 | trace amount |
| Example 13 | 4.42 | none | 3.0 | 0.55 | 31 | 85 | 26 | trace amount | trace amount |
| Comparative Example 1 | 401 | 100 | 3.0 | 2.2 | >99 | 39 | 38 | 20 | 33 |

(continued)

| | BET [m²/g] | Crystallinity [%] | Raw material molar ratio | WHSV [h⁻¹] | MeOH conversion rate [%] | MeNH₂ selectivity [%] | MeNH₂ yield [%] | Me₂NH yield [%] | Me₃N yield [%] |
|---|---|---|---|---|---|---|---|---|---|
| Comparative Example 2 | 349 | 100 | 3.0 | 1.1 | 79 | 73 | 57 | 4.3 | 8.1 |
| Comparative Example 3 | 68.6 | 99.4 | 3.0 | 0.55 | 91 | 17 | 15 | 18 | 52 |

(Example 2)

Monomethylamine was produced in the same manner as in Example 1, except that the ammonia flow rate was 1.0 L/hr, the methanol flow rate was 0.55 mL/hr, and the weight hourly space velocity (WHSV) was 1.1/hr.

(Example 3)

Monomethylamine was produced in the same manner as in Example 1, except that the raw material molar ratio (ammonialmethanol) was 1.0 by setting the methanol flow rate to 0.81 mL/hour, the ammonia feed amount was 0.21 g, and the weight hourly space velocity (WHSV) was 0.98/hour.

(Example 4)

Monomethylamine was produced in the same manner as in Example 1, except that the raw material molar ratio (ammonialmethanol) was 0.50 by setting the methanol flow rate to 1.6 mL/hour, the ammonia feed amount was 0.11 g, and the weight hourly space velocity (WHSV) was 1.6/hour.

(Example 5)

A solid catalyst of Example 5 was produced in the same manner as in Example 1, except that 36.4 mL of the solution obtained by dissolving a basic salt in a solvent which is the same as that of Example 1 were mixed with 3.0 g of 620HOA, which is the raw material mordenite. In Example 5, the ratio of the amount of cations in the basic salt contained in the solution to the amount of points in which cations can be provided in the raw material mordenite was 2.0.
Regarding the obtained solid catalyst, the specific surface area and the crystallinity were determined in the same manner as in Example 1. As a result, as shown in Table 1, the specific surface area of the solid catalyst of Example 5 was 12.5 $m^2$/g, and the crystallinity was 94.8%.
Thereafter, monomethylamine was produced in the same manner as in Example 1 except that the solid catalyst of Example 5 was used.

(Example 6)

0.40 g of sodium hydroxide was dissolved in pure water, and the volume was adjusted to 50 mL using a volumetric flask to prepare a solution obtained by dissolving the basic salt in a solvent (0.20 mol/L). The entire amount of the obtained aqueous solution and 4.5 g of 620HOA, which is the raw material mordenite, were mixed (mixing step). Thereafter, a solid catalyst of Example 6 was produced in the same manner as in Example 1. In Example 6, the ratio of the amount of cations in the basic salt contained in the solution to the amount of points in which cations can be provided in the raw material mordenite was 1.1.
Regarding the obtained solid catalyst, the specific surface area and the crystallinity were determined in the same manner as in Example 1. As a result, as shown in Table 1, the specific surface area of the solid catalyst of Example 6 was 16.5 $m^2$/g, and the crystallinity was 95.6%.
Thereafter, monomethylamine was produced in the same manner as in Example 1 except that the solid catalyst of Example 6 was used.

(Example 7)

3.1 g of sodium carbonate ($Na_2CO_3$, manufactured by Junsei Kagaku Co., Ltd.) was dissolved in pure water, and the volume was adjusted to 100 mL using a volumetric flask to prepare a solution obtained by dissolving the basic salt in a solvent. A solid catalyst of Example 7 was produced in the same manner as in Example 1 except that the entire amount of the obtained aqueous solution was used in the mixing step. In Example 7, the ratio of the amount of cations in the basic salt contained in the solution to the amount of points in which cations can be provided in the raw material mordenite was 1.9.
Regarding the obtained solid catalyst, the specific surface area and the crystallinity were determined in the same manner as in Example 1. As a result, as shown in Table 1, the specific surface area of the solid catalyst of Example 7 was 6.2 $m^2$/g, and the crystallinity was 93.5%.
Thereafter, monomethylamine was produced in the same manner as in Example 1 except that the solid catalyst of Example 7 was used.

(Example 8)

A solid catalyst was produced using the second production method.

That is, 9.0 g of sodium hydroxide was dissolved in pure water, and the volume was adjusted to 150 mL using a volumetric flask to prepare a solution obtained by dissolving the basic salt in a solvent. 132 mL of the obtained aqueous solution was measured and mixed with 3.0 g of 620HOA, which is the raw material mordenite, in a glass container to obtain a mixed liquid (mixing step). In Example 8, the ratio of the amount of cations in the basic salt contained in the solution to the amount of points in which cations can be provided in the raw material mordenite was 33.

Thereafter, the mixed liquid was stirred at room temperature for 12 hours to obtain a solution-treated mordenite (stirring step). The solution-treated mordenite was collected from the mixture by suction filtration and cleaned with water. The mordenite cleaned with water and treated with a solution was transferred to a magnetic crucible, and dry-fired in an electric furnace set at 500°C for 12 hours in an air atmosphere to obtain a solid catalyst of Example 8 (firing step).

Regarding the obtained solid catalyst, the specific surface area and the crystallinity were determined in the same manner as in Example 1. As a result, as shown in Table 1, the specific surface area of the solid catalyst of Example 8 was 17.0 $m^2/g$, and the crystallinity was 100%.

Although the crystallinity of the solid catalyst of Example 8 was 100%, it is only 100% in significant figures, and it is estimated that the value is actually slightly smaller than 100%. That is, in Example 8, in the stirring step, a reaction between the aqueous sodium hydroxide solution and the raw material mordenite was carried out, some components of the raw material mordenite were dissolved, and sodium ions were adsorbed (supported) on the raw material mordenite; and then, in the firing step, it is presumed that a very thin surface layer with micropores is formed.

Thereafter, monomethylamine was produced in the same manner as in Example 1 except that the solid catalyst of Example 8 was used.

(Example 9)

1.2 g of sodium hydroxide was dissolved in pure water, and the volume was adjusted to 100 mL using a volumetric flask to prepare a solution obtained by dissolving the basic salt in a solvent. 31 mL of the obtained aqueous solution was measured and mixed with 3.0 g of 620HOA, which is the raw material mordenite (mixing step). Thereafter, a solid catalyst of Example 9 was produced in the same manner as in Example 1. In Example 9, the ratio of the amount of cations in the basic salt contained in the solution to the amount of points in the raw material mordenite to which cations can be provided was 1.5.

Regarding the obtained solid catalyst, the specific surface area was determined in the same manner as in Example 1. As a result, as shown in Table 1, the specific surface area of the solid catalyst of Example 9 was 53.8 $m^2/g$.

Thereafter, monomethylamine was produced in the same manner as in Example 1 except that the solid catalyst of Example 9 was used.

(Example 10)

0.43 g of sodium hydroxide was dissolved in 4.5 mL of pure water to prepare a solution obtained by dissolving the basic salt in a solvent. The entire amount of the obtained aqueous solution and 5.0 g of 620HOA, which is the raw material mordenite, were mixed (mixing step). Thereafter, a solid catalyst of Example 10 was produced in the same manner as in Example 1. In Example 10, the ratio of the amount of cations in the basic salt contained in the solution to the amount of points in which cations can be provided in the raw material mordenite was 1.1.

Regarding the obtained solid catalyst, the specific surface area was determined in the same manner as in Example 1. As a result, as shown in Table 1, the specific surface area of the solid catalyst of Example 10 was 27.0 $m^2/g$.

Thereafter, monomethylamine was produced in the same manner as in Example 1 except that the solid catalyst of Example 10 was used.

(Example 11)

0.62 g of sodium carbonate was dissolved in 4.0 mL of pure water to prepare a solution obtained by dissolving the basic salt in a solvent. The entire amount of the obtained aqueous solution and 5.0 g of 620HOA, which is the raw material mordenite, were mixed (mixing step). Thereafter, a solid catalyst of Example 11 was produced in the same manner as in Example 1. In Example 11, the ratio of the amount of cations in the basic salt contained in the solution to the amount of points in which cations can be provided in the raw material mordenite was 1.2.

Regarding the obtained solid catalyst, the specific surface area was determined in the same manner as in Example 1. As a result, as shown in Table 1, the specific surface area of the solid catalyst of Example 11 was 32.4 $m^2/g$.

Thereafter, monomethylamine was produced in the same manner as in Example 1 except that the solid catalyst of Example 11 was used.

(Example 12)

A solid catalyst of was produced using the third production method.
The solid catalyst of Example 12 was produced by firing 5.0 g of a basic mordenite having $Na^+$ as a cation, which is the raw material mordenite, at 700°C for 1 hour (high-temperature firing step).
Regarding the obtained solid catalyst, the specific surface area was determined in the same manner as in Example 1. As a result, as shown in Table 1, the specific surface area of the solid catalyst of Example 12 was 9.37 $m^2$/g.
Thereafter, monomethylamine was produced in the same manner as in Example 1 except that the solid catalyst of Example 12 was used.

(Example 13)

2.3 g of sodium hydroxide was dissolved in pure water, and the volume was adjusted to 200 mL using a volumetric flask to prepare a solution obtained by dissolving the basic salt in a solvent. The entire amount of the obtained aqueous solution and 15 g of 620HOA, which is the raw material mordenite, were mixed (mixing step). Thereafter, a solid catalyst of Example 13 was produced in the same manner as in Example 1. In Example 13, the ratio of the amount of cations in the basic salt contained in the solution to the amount of points in which cations can be provided in the raw material mordenite was 1.9.
Regarding the obtained solid catalyst, the specific surface area was determined in the same manner as in Example 1. As a result, as shown in Table 1, the specific surface area of the solid catalyst of Example 13 was 4.42 $m^2$/g.
Thereafter, monomethylamine was produced in the same manner as in Example 1 except that the solid catalyst of Example 13 was used.

(Comparative Example 1)

Monomethylamine was produced in the same manner as in Example 1, except that 620HOA was used as the solid catalyst, the ammonia flow rate was 2.0 L/hour, the methanol flow rate was 1.1 mL/hour, and the weight hourly space velocity (WHSV) was 2.2/hour.
Regarding the catalyst of Comparative Example 1, the specific surface area and the crystallinity were determined in the same manner as in Example 1. As a result, the specific surface area of the solid catalyst of Comparative Example 1 was 401 $m^2$/g, and the crystallinity was 100%.

(Comparative Example 2)

Monomethylamine was produced in the same manner as in Example 1, except that a basic mordenite having $Na^+$ as a cation (trade name: 642NAA, manufactured by Tosoh Corporation) was used as the solid catalyst, and the weight hourly space velocity (WHSV) was 1.1 per hour.
Regarding the catalyst of Comparative Example 2, the specific surface area and the crystallinity were determined in the same manner as in Example 1. As a result, the specific surface area of the solid catalyst of Comparative Example 1 was 349 $m^2$/g, and the crystallinity was 100%.

(Comparative Example 3)

A mixing step was performed using the entire amount of an aqueous solution which was obtained by was dissolving 4.2 g of sodium sulfate ($Na_2SO_4$, manufactured by Junsei Kagaku Co., Ltd.) in pure water and adjusting the volume to 100 mL using a volumetric flask, instead of the solution obtained by dissolving the basic salt in a solvent. Thereafter, a solid catalyst of Comparative Example 3 was produced in the same manner as in Example 1.
Regarding the obtained solid catalyst, the specific surface area and the crystallinity were determined in the same manner as in Example 1. As a result, as shown in Table 1, the specific surface area of the solid catalyst of Comparative Example 3 was 68.6 $m^2$/g, and the crystallinity was 99.4%.
Thereafter, monomethylamine was produced in the same manner as in Example 1 except that the solid catalyst of Comparative Example 3 was used.
The compositions of the reaction products obtained by the reactions of Examples 2 to 13 and Comparative Examples 1 to 3 were analyzed using gas chromatography (GC).
As a result, in all of Examples 2 to 13 and Comparative Examples 1 to 3, monomethylamine, which is the target product, was detected from the reaction products, and dimethylamine, trimethylamine, and dimethyl ether were also detected as by-products. The unreacted raw materials, ammonia and methanol, were also detected.

In the reactions of Examples 2 to 13 and Comparative Examples 1 to 3, each conversion rate of methanol (MeOH), selectivity of monomethylamine (MeNH$_2$), and each yield of monomethylamine (MeNH$_2$), dimethylamine (Me$_2$NH), and trimethylamine (Me$_3$N) were determined in the same manner as in Example 1. The results are shown in Table 1.

The solid catalysts of Examples 1 to 13 were produced using a raw material mordenite, as shown in Table 1. The specific surface area (BET) calculated by the BET method was 4 to 60 m$^2$/g, and the crystal structure was a mordenite-type zeolite. By reacting ammonia and methanol in the presence of the solid catalysts of Examples 1 to 13, as shown in Table 1, it is possible to ensure a conversion rate of methanol of 30% or more, the amount of trimethylamine was a trace amount, and monomethylamine was obtained with a high selectivity of 75% or more.

From this, in the solid catalysts of Examples 1 to 13, micropores were formed through which monomethylamine molecules can pass, but through which trimethylamine molecules cannot pass, during the process of producing the solid catalysts using the raw material mordenite. It is estimated that in Examples 1 to 6, Examples 8 to 10, and Example 13, in which sodium hydroxide was used as a basic salt in the process of producing the solid catalyst, the smaller the specific surface area, the higher the probability that the formation of dimethylamine was suppressed.

In addition, the reaction of Example 1 had higher and better methanol conversion, monomethylamine yield, and monomethylamine selectivity than Example 2, which used the same solid catalyst and had a different weight hourly space velocity (WHSV).

In addition, the reaction of Example 1 had higher and better methanol conversion, monomethylamine yield, and monomethylamine selectivity than Examples 3 and 4, which used the same solid catalyst and had different raw material molar ratios (ammonialmethanol) and weight hourly space velocities (WHSV).

From Examples 1, 5, 6, 9, 10, and 13, it was confirmed that solid catalysts with different specific surface areas can be produced by changing the ratio of the amount of cations in the basic salt contained in the solution to the amount of points in which cations can be provided in the raw material mordenite used in the mixing step ((total of [valence $\times$ amount] of cations in the salt contained in the solution)/(amount of points in which cations can be provided in the raw material mordenite)), and/or the concentration of the basic salt contained in the solution obtained by dissolving the basic salt in a solvent.

In the reaction of Comparative Example 1 in which the raw material mordenite in Example 1 was used as a solid catalyst, monomethylamine, dimethylamine, and trimethylamine were produced. This is because monomethylamine is produced by the above-mentioned (first reaction), and the electron density on the nitrogen atom increases due to the addition of a methyl group, which is an electron donating group, and the (second reaction) and (third reaction)) has progressed.

As a result, in the reaction of Comparative Example 1, the yield of trimethylamine was as high as 33%. Furthermore, in the reaction of Comparative Example 1, the selectivity of monomethylamine was 39%, which was lower than that of Examples 1 to 13.

The solid catalyst used in Comparative Example 2 has Na$^+$ as a cation, and the acid sites are partially neutralized. Therefore, in the reaction of Comparative Example 2 using this solid catalyst, the reaction rates in the above-mentioned (first reaction) to (fourth reaction) are slower than in Comparative Example 1. Therefore, in Comparative Example 2, even though the weight hourly space velocity (WHSV) was made smaller and the contact time between the solid catalyst and the raw material was longer than in Comparative Example 1, the yield and the selectivity of monomethylamine were high and the yields of dimethylamine and trimethylamine were low.

The solid catalyst of Comparative Example 3 had a specific surface area (BET), calculated by the BET method, of 68.6 m$^2$/g, exceeding 60 m$^2$/g, because a neutral sodium sulfate aqueous solution was used in the mixing step instead of a solution obtained by dissolving the basic salt in a solvent. It is presumed that the result is due to the fact that the surface of the raw material mordenite was not sufficiently dissolved in the solid catalyst producing process. Therefore, it is presumed that a surface layer having micropores through which monomethylamine molecules can pass but through which trimethylamine molecules cannot pass was not formed on the surface of the solid catalyst of Comparative Example 3. As a result, it is presumed that in the reaction using the solid catalyst of Comparative Example 3, the production of trimethylamine could not be suppressed, and the selectivity of monomethylamine decreased.

(Reference Example 1)

Monoethylamine was produced in the same manner as in Example 1, except that ethanol was used instead of methanol, and the feed amounts of ammonia and ethanol were continued until they reached 0.55 g and 0.50 g, respectively.

The composition of the reaction product obtained by the reaction of Reference Example 1 was analyzed using gas chromatography (GC).

As a result, in Reference Example 1, the reaction product contained monoethylamine, diethylamine, triethylamine, ethanol, and diethyl ether produced by the reaction between ethanols, and many other organic substances were detected.

Furthermore, the yield of monoethylamine was determined in the same manner as the yield of monomethylamine in Example 1. As a result, the yield of monoethylamine was 0.4%.

It is presumed that ethanol could not come into contact with the reaction points existing in the pores derived from the raw material mordenite which was used as a raw material for the catalyst, because ethanol has a large molecular size, and could not pass through the surface layer which was formed on the solid catalyst of Example 1, wherein the surface layer has micropores through which monomethylamine molecules can pass but through which trimethylamine molecules cannot pass. It is considered that the ethanol that could not come into contact with the reaction points of the solid catalyst in Example 1 was not promoted to react with ammonia and was used for multiple types of reactions outside the solid catalyst.

**Claims**

1. A method for producing a solid catalyst which has a specific surface area of 4 to 60 $m^2/g$ and is a mordenite-type zeolite, the method comprising:

   a mixing step of mixing a raw material mordenite and a solution obtained by dissolving a basic salt in a solvent to form a mixed liquid;
   an evaporating-drying step of evaporating the mixed liquid to dryness to obtain a solution-treated mordenite; and
   a firing step of firing the solution-treated mordenite.

2. The method for producing a solid catalyst according to claim 1, wherein the solid catalyst has a crystallinity of 80% or more.

3. The method for producing a solid catalyst according to claim 1 or 2, wherein the solid catalyst is a solid catalyst for producing monomethylamine via a reaction between ammonia and a methylating agent.

4. The method for producing a solid catalyst according to claim 1 or 2,
   wherein the firing step comprises

   a first firing step of obtaining fired mordenite by firing the solution-treated mordenite;
   a cleaning step of cleaning the fired mordenite with the solvent; and
   a second firing step of firing the cleaned fired mordenite.

5. The method for producing a solid catalyst according to claim 1 or 2, wherein the basic salt comprises a sodium ion as a cation.

6. The method for producing a solid catalyst according to claim 1 or 2, wherein the basic salt comprises at least one selected from the group consisting of hydroxide ions and carbonate ions as anions.

7. The method for producing a solid catalyst according to claim 1 or 2, wherein the basic salt comprises sodium hydroxide.

8. A method for producing a solid catalyst which has a specific surface area of 4 to 60 $m^2/g$ and is a mordenite-type zeolite, the method comprising:

   a mixing step of mixing a raw material mordenite and a solution obtained by dissolving a basic salt in a solvent to form a mixed liquid;
   a stirring step of stirring the mixed liquid to obtain a solution-treated mordenite; and
   a firing step of firing the solution-treated mordenite.

9. The method for producing a solid catalyst according to claim 8, wherein the solid catalyst has a crystallinity of 80% or more.

10. The method for producing a solid catalyst according to claim 8 or 9, wherein the solid catalyst is a solid catalyst for producing monomethylamine via a reaction between ammonia and a methylating agent.

**11.** The method for producing a solid catalyst according to claim 8 or 9, wherein the basic salt comprises a sodium ion as a cation.

**12.** The method for producing a solid catalyst according to claim 8 or 9, wherein the basic salt comprises a hydroxide ion as an anion.

**13.** The method for producing a solid catalyst according to claim 8 or 9, wherein the basic salt comprises sodium hydroxide.

**14.** A method for producing a solid catalyst which has a specific surface area of 4 to 60 $m^2/g$ and is a mordenite-type zeolite, the method comprising:
a firing step of firing a raw material mordenite at a temperature of 650°C to 1000°C.

**15.** The method for producing a solid catalyst according to claim 14, wherein the solid catalyst has a crystallinity of 80% or more.

**16.** The method for producing a solid catalyst according to claim 14 or 15, wherein the solid catalyst is a solid catalyst for producing monomethylamine via a reaction between ammonia and a methylating agent.

**17.** A solid catalyst which has a specific surface area of 4 to 60 $m^2/g$ and is a mordenite-type zeolite, wherein the solid catalyst is produced by the method for producing a solid catalyst according to any one of claims 1, 8, and 14.

**18.** A method for producing monomethylamine, the method comprising:
reacting ammonia and a methylating agent in the presence of the solid catalyst according to claim 17.

**19.** The method for producing monomethylamine according to claim 18, wherein the methylating agent is methanol.

**20.** The method for producing monomethylamine according to claim 18, wherein a molar ratio of an amount of ammonia used to an amount of the methylating agent used is within a range of 0.2 to 5.

**21.** The method for producing monomethylamine according to claim 18, wherein a weight hourly space velocity in the reaction is within a range of 0.1 to 3.0 per hour.

## INTERNATIONAL SEARCH REPORT

| International application No. |
|---|
| **PCT/JP2022/037566** |

**A.     CLASSIFICATION OF SUBJECT MATTER**

***B01J 29/18***(2006.01)i; ***B01J 35/10***(2006.01)i; ***B01J 37/04***(2006.01)i; ***B01J 37/06***(2006.01)i; ***B01J 37/08***(2006.01)i;
***C01B 39/26***(2006.01)i; ***C07B 61/00***(2006.01)i; ***C07C 209/14***(2006.01)i; ***C07C 211/04***(2006.01)i
FI:    B01J29/18 Z; C07C211/04; C07C209/14; B01J37/04 102; B01J37/08; B01J37/06; B01J35/10 301J; B01J35/10 301G;
C07B61/00 300; C01B39/26

According to International Patent Classification (IPC) or to both national classification and IPC

**B.     FIELDS SEARCHED**

Minimum documentation searched (classification system followed by classification symbols)
B01J21/00-38/74; C07C1/00-409/44;C07B61/00;C01B39/26

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JST7580/JSTChina (JDreamIII);CAplus (STN)

**C.     DOCUMENTS CONSIDERED TO BE RELEVANT**

| Category* | Citation of document, with indication, where appropriate, of the relevant passages | Relevant to claim No. |
|---|---|---|
| X | JP 6-238169 A (SEKIYU SANGYO KASSEIKA CENTER) 30 August 1994 (1994-08-30) claim 1, example 7 | 14, 15, 17 |
| A | | 1-13, 16, 18-21 |
| X | JP 8-193057 A (MITSUI TOATSU CHEM INC) 30 July 1996 (1996-07-30) claim 1, paragraphs [0001], [0037], examples 2, 3, 12, tables 1, 2 | 14-21 |
| A | | 1-13 |
| A | JP 2012-526787 A (BP CHEMICALS LIMITED) 01 November 2012 (2012-11-01) claim 1, example 1, table 1 | 1-21 |
| A | JP 59-227841 A (NITTO CHEMICAL INDUSTRY CO LTD) 21 December 1984 (1984-12-21) claim 1, example 7 | 1-21 |
| A | JP 8-225498 A (NITTO CHEMICAL INDUSTRY CO LTD) 03 September 1996 (1996-09-03) claim 1, examples 1-13 | 1-21 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **24 November 2022** | **06 December 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2022/037566**

| Patent document<br>cited in search report | | | Publication date<br>(day/month/year) | Patent family member(s) | | | Publication date<br>(day/month/year) |
|---|---|---|---|---|---|---|---|
| JP | 6-238169 | A | 30 August 1994 | (Family: none) | | | |
| JP | 8-193057 | A | 30 July 1996 | (Family: none) | | | |
| JP | 2012-526787 | A | 01 November 2012 | WO | 2010/130973 | A2 | |
| | | | | claim 1, example 1, table 1 | | | |
| | | | | US | 2012/0053360 | A1 | |
| | | | | EP | 2251082 | A1 | |
| | | | | KR | 10-2012-0020120 | A | |
| JP | 59-227841 | A | 21 December 1984 | US | 4582936 | A | |
| | | | | claim 1, example 7 | | | |
| | | | | EP | 130407 | A1 | |
| | | | | KR | 10-1985-0000383 | A | |
| JP | 8-225498 | A | 03 September 1996 | US | 5773659 | A | |
| | | | | claim 1, examples 1-13 | | | |
| | | | | WO | 1996/017820 | A1 | |
| | | | | EP | 744395 | A1 | |
| | | | | CN | 1140446 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021166985 A
- JP 3995611 B
- JP 4758645 B

- JP H0216743 B
- JP 63025575 A
- JP 2007161637 A

**Non-patent literature cited in the description**

- Complete collection of revised manufacturing process diagrams. Kagaku Kogyosha Co., Ltd, 25 April 1978, 440-441